# EUROPÄISCHE PATENTANMELDUNG

(11) **EP 0 890 357 A2**
(43) Veröffentlichungstag der Anmeldung: **13.01.1999**
(21) Anmeldenummer: 98109960.9
(22) Anmeldetag: 02.06.1998
(51) Int. Cl.: A61K 7/50, A61K 7/48

(54) **Kosmetische und/oder pharmazeutische Zubereitungen**

(30) Priorität: 12.06.1997 DE 19724867
(71) Anmelder: Henkel Kommanditgesellschaft auf Aktien, 40589 Düsseldorf-Holthausen (DE)
(72) Erfinder: Bigorra, Joaquin, Dr., 08203 Sabadell (ES); Prat Queralt, Ester, Dr., 02838 Alella (ES); Pi Subirana, Rafael, Dr., 08400 Granollers (ES)

(57) **Zusammenfassung**

Vorgeschlagen werden kosmetische und/oder pharmazeutische Zubereitungen, enthaltend
(a) Methylmischether und
(b) Esterquats.

Methylmischether weisen ausgezeichnete emulgierende Eigenschaften auf. In Abmischung mit Esterquats verleihen sie Emulsionen avivierende und konditionierende Eigenschaften sowie eine ausreichend hohe und auch bei höheren Temperaturen konstante Stabilität.

## Beschreibung

### Gebiet der Erfindung

Die Erfindung betrifft kosmetische bzw. pharmazeutische Zubereitungen, mit einem Gehalt an einer Emulgatormischung aus Methylmischethern und Esterquats sowie die Verwendung von Methylmischethern als Emulgatoren zur Herstellung dieser Zubereitungen.

### Stand der Technik

Kosmetische Produkte wie beispielsweise Cremes oder Lotionen, aber auch pharmazeutische Zubereitungen wie etwa Salben stellen aus physikochemischer Sicht in der Regel Emulsionen, vorzugsweise O/W-Emulsionen dar. Zur Vermischung der wäßrigen und der öligen Phase werden Emulgatoren benötigt, von denen eine schier unerschöpfliche Vielfalt in der Literatur beschrieben werden, auch wenn nur wenige Gruppen wirklich technische Bedeutung besitzen. Obschon eine Emulsion schon aus thermodynamischer Sicht nur ein metastabiler Sonderfall eines ansonsten zweiphasigen Gemisches darstellt, geht doch das Bestreben des Kosmetikchemikers dahin, diesen Zustand solange wie möglich aufrechtzuerhalten. Demzufolge besteht im Markt ein ständiges Bedürfnis nach hautkosmetisch besonders verträglichen Emulgatoren, die Emulsionen über einen möglichst langen Zeitraum stabilisieren. Eine weitere Anforderung an derartige Emulgatoren besteht darin, den Emulsionen auch eine ausreichend hohe und vor allem bei Lagerung konstante Viskosität zu verleihen. Diese Eigenschaften werden des weiteren vor allem auch dann verlangt, wenn die Lagerung bei erhöhten Temperaturen erfolgt. Es ist sofort klar, daß eine Verbraucherin, deren wertvolle Pflegecreme sich bei Sonneneinstrahlung irreversibel in eine dünnflüssige zweiphasige Mischung verwandelt, diese Kaufentscheidung kein zweites Mal treffen wird. Die Aufgabe der Erfindung hat demnach darin bestanden, Emulsionen zur Verfügung zu stellen, die frei von den geschilderten Nachteilen sind und darüber hinaus nach Möglichkeit weitere pflegende Eigenschaften besitzen.

In diesem Zusammenhang sei darauf hingewiesen, daß aus der **EP-A 0161537** (BASF) die Verwendung u.a. von methylendgruppenverschlossenen Mischethern für die maschinelle Flaschenreinigung bekannt ist. In der **EP-B 0180081** (BASF) werden die gleichen Stoffe als Entschäumer für die Zucker- und Hefeindustrie vorgeschlagen. Gegenstand der **EP-B 0202638** (BASF) sind schließlich flüssige Tensidkonzentrate für stark alkalische Reinigungsmittel, enthaltend Methylmischether, Alkylpolyglucoside, Anlagerungsprodukte von Maleinsäureanhydrid an ungesättigte Fettsäuren sowie verzweigte Fettsäuren.

### Beschreibung der Erfindung

Gegenstand der Erfindung sind kosmetische und/oder pharmazeutische Zubereitungen, enthaltend
(a) Methylmischether und
(b) Esterquats.

Überraschenderweise wurde gefunden, daß die bislang nur als schaumarme Netzmittel bekannten Methylmischether ausgesprochen hautverträglich sind und über ausgezeichnete emulgierende Eigenschaffen verfügen. Unter Verwendung dieser Stoffe lassen sich stabile Emulsionen herstellen, die eine ausreichend hohe Viskosität besitzen und diese auch bei Temperaturbelastung im wesentlichen beibehalten. Die Erfindung schließt die Erkenntnis ein, daß Mischungen der Methylmischether mit kationischen Tensiden vom Esterquattyp zu einem weiteren vorteilhaften Anstieg der Viskosität führt und die Stabilität der Emulsionen weiter verbessert. Gleichzeitig zeichnen sich derartige Zubereitungen durch ausgezeichnete avivierende Eigenschaffen aus, was sie besonders interessant für die Herstellung von Haut- und Haarpflegemittel macht.

### Methylmischether

Methylmischether stellen bekannte Stoffe dar, die durch Alkylierung von Alkoholpolyglycolethern mit Methylhalogeniden, vorzugsweise aber Dialkylsulfat hergestellt werden. Einschlägige Verfahren sind beispielsweise aus den Druckschriften **US 4,587,365, EP-B 0302487** (BASF) sowie den beiden internationalen Patentanmeldungen **WO 96/14 377** und **WO 96/21 636** (Henkel) bekannt. In einer bevorzugten Ausführungsform der Erfindung folgen die Methylmischether der Formel **(I)**, 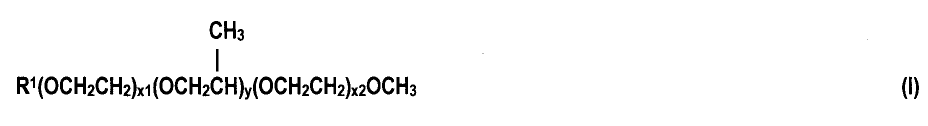 in der R¹ für einen linearen oder verzweigten Alkyl- und/oder Alkenylrest mit 6 bis 22, vorzugsweise 16 bis 18 Kohlenstoffatomen, x1 und x2 unabhängig voneinander für 0 oder Zahlen von 1 bis 50, vorzugsweise 10 bis 40 und insbesondere 15 bis 25, y für 0 oder Zahlen von 1 bis 5 steht, mit der Maßgabe, daß x und y nicht gleichzeitig für 0 stehen können. Typische Beispiele sind Methylierungsprodukte von Addukten von 1 bis 50, vorzugsweise 5 bis 40 und insbesondere 15 bis 25 Mol Ethylenoxid und/oder 1 bis 5 Mol Propylenoxid - in Random- oder Blockverteilung - an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Umsetzungsprodukte von Cetearylalkohol+10EO, Cetearylakohol+2PO+10EO, Cetearylalkohol+20EO, Cetearylalkohol+30EO, Stearylalkohol+40EO, Isostearylalkohol+40EO sowie deren Mischungen mit Methylchlorid oder Dimethylsulfat.

### Esterquats

Unter der Bezeichnung 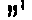 Esterquats" werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der Deutschen Patentschrift **DE-C1 4308794** (Henkel) ist überdies ein Verfahren zur Herstellung fester Esterquats bekannt, bei dem man die Quaternierung von Triethanolaminestern in Gegenwart von geeigneten Dispergatoren, vorzugsweise Fettalkoholen, durchführt. Übersichten zu diesem Thema sind beispielsweise von R.Puchta et al. in **Tens.Surf.Det., 30, 186 (1993),** M.Brock in **Tens.Surf.Det. 30, 394 (1993),** R.Lagerman et al. in **J.Am.Oil.Chem.Soc., 71, 97 (1994)** sowie I.Shapiro in **Cosm.Toil. 109, 77 (1994)** erschienen.

Die quaternierten Fettsäuretriethanolaminestersalze folgen der Formel **(II),** 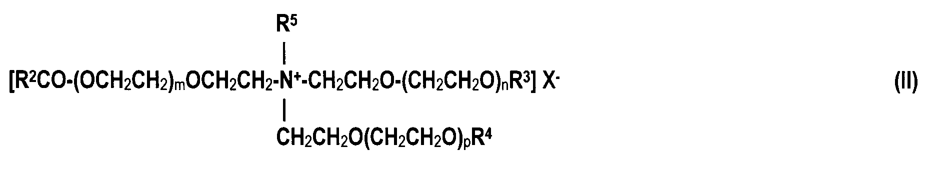 in der R²CO für einen Acylrest mit 6 bis 22 Kohlenstoffatomen, R³ und R⁴ unabhängig voneinander für Wasserstoff oder R²CO, R⁵ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}- Talg- bzw. Palmfettsäure (Iodzahl 0 bis 40) ab. Aus anwendungstechnischer Sicht haben sich quaternierte Fettsäuretriethanolaminestersalze der Formel **(II)** als besonders vorteilhaft erwiesen, in der R¹CO für einen Acylrest mit 16 bis 18 Kohlenstoffatomen, R³ für R²CO, R⁴ für Wasserstoff, R⁵ für eine Methylgruppe, m, n und p für 0 und X für Methylsulfat steht.

Neben den quaternierten Fettsäuretriethanolaminestersalzen kommen als Esterquats ferner auch quaternierte Estersalze von Fettsäuren mit Diethanolalkylaminen der Formel **(III)** in Betracht, 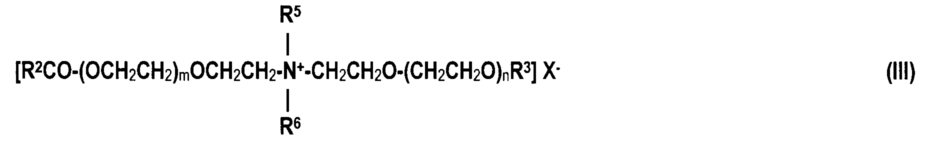 in der R²CO für einen Acylrest mit 6 bis 22 Kohlenstoffatomen, R³ für Wasserstoff oder R²CO, R⁵ und R⁶ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel **(IV)** zu nennen, 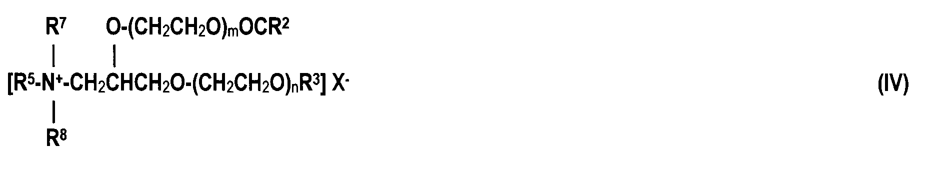 in der R²CO für einen Acylrest mit 6 bis 22 Kohlenstoffatomen, R³ für Wasserstoff oder R²CO, R⁵, R⁷ und R⁸ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für **(II)** genannten Beispiele auch für die Esterquats der Formeln **(III)** und **(IV)**. Üblicherweise gelangen die Esterquats in Form 50 bis 90 Gew.-%iger alkoholischer Lösungen in den Handel, die bei Bedarf problemlos mit Wasser verdünnt werden können.

### Co-Emulgatoren

Als Co-Emulgatoren kommen beispielsweise nichtionogene Tenside aus mindestens einer der folgenden Gruppen in Frage:
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxid-anlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Lauryl-glucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind hinlänglich bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Der Anteil der Co-Emulgatoren kann bezogen auf die Emulgatoren 5 bis 50, vorzugsweise 10 bis 40 und insbesondere 15 bis 25 Gew.-% ausmachen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Dialkylether, Ringöffnungsprodukte von epoxidierten Fettsäurealkylestern mit Polyolen, Siliconöle und/oder aliphatische bzw. naphthenische Kohlenwasserstoffe in Betracht. Der Anteil der Ölkörper an den Zubereitungen kann 10 bis 90, vorzugsweise 15 bis 70 und insbesondere 25 bis 50 Gew.-% - bezogen auf die Mittel - betragen.

### Gewerbliche Anwendbarkeit

Die Erfindung schließt die Erkenntnis ein, daß Methylmischether, die bislang nur als schaumarme Netzmittel bekannt waren, über ausgezeichnete emulgierende Eigenschaften verfügen. Dementsprechend betrifft ein weiterer Gegenstand der Erfindung die Verwendung von Methylmischethern nach Anspruch 2 als Emulgatoren zur Herstellung von kosmetischen und/oder pharmazeutischen Zubereitungen, in denen sie in Mengen von 0,1 bis 10, vorzugsweise 1 bis 5 Gew.-% - bezogen auf die Mittel - enthalten sein können.

### Kosmetische und/oder pharmazeutische Zubereitungen

Die erfindungsgemäßen Zubereitungen, wie beispielsweise Haarshampoos, Haarlotionen, Schaumbäder, Cremes, Lotionen oder Salben, können ferner als weitere Hilfs- und Zusatzstoffe milde Tenside, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Farb- und Duftstoffe enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quaternierte Hydroxyethylcellulose, die unter der Bezeichnung Polymer JR 400® von Amerchol erhältich ist, kationische Stärke, Copolymere von Diallylammoniumsalzen und Acrylamiden, quaternierte Vinylpyrrolidon/Vinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentrimamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quaternierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor-, glucosid- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherol-acetat, Tocopherolpalmitat, Ascorbinsäure, Retinol, Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinyl-pyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage als **Selbstbräuner** eignet sich Dihydroxyaceton. Als **Farbstoffe** können die für kosmetische Zwecke geeigneten und zugelassenen Substanzen verwendet werden, wie sie beispielsweise in der Publikation **''Kosmetische Färbemittel'' der Farbstoffkommission der Deutschen Forschungsgemeinschaff, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

**Beispiele 1 bis 4, Vergleichsbeispiele V1 bis V4.** Die Viskosität 4 Gew.-%iger wäßriger Zubereitungen verschiedener Methylmischether und Fettalkoholpolyglycolether wurde nach der Brookfleld Methode in einem RVT-Viskosimeter (20/40°C, 10 Upm) sofort sowie nach einer Lagerzeit von 4 Wochen bestimmt. Die Lagerstabilität wurde optisch bestimmt. Dabei bedeutet (+) einphasig und (-) getrennt. Die Ergebnisse sind in Tabelle 1 zusammengefaßt. Die Beispiele 1 bis 4 sind erfindungsgem**ä**ße die Beispiele V1 bis V4 dienen zum Vergleich.

**Tabelle 1**

| **Viskositätsmessungen** | | | | | |
|---|---|---|---|---|---|
| **Bsp.** | **Emulgator** | **Viskostät** | | **Stabilität** | |
| | | **sofort, 20°C** | **4 w, 40°C** | **sofort, 20°C** | **4 w, 40°C** |
| 1 | Cetearylalkohol+7EO-Methylether | 7.000 | 6.500 | + | + |
| 2 | Cetearylalkohol+11EO-Methylether | 8.000 | 7.500 | + | + |
| 3 | Cetearylalkohol+20EO-Methylether | 11.000 | 10.500 | + | + |
| 4 | Cetearylalkohol+40EO-Methylether | 6.000 | 5.500 | + | + |
| V1 | Cetearylalkohol+7EO | 5.000 | - | + | - |
| V2 | Cetearylalkohol+11EO | 7.000 | - | + | - |
| V3 | Cetearylalkohol+20EO | 9.000 | 4.500 | + | + |
| V4 | Cetearylalkohol+40EO | 3.000 | 1.000 | + | + |

Die Beispiele und Vergleichsbeispiele zeigen, daß die methylendgruppenverschlossenen Fettalkoholpolyglycolether den offenkettigen Vergleichsprodukten bezüglich Viskosität und Viskositätsstabilität deutlich überlegen sind.

**Beispiele 5 bis 7, Vergleichsbeispiele V5 bis V7.** Der Einfluß von Emulgatoren auf die Viskosität und Viskositätsstabilität verschiedener Emulsionen wurde nach der Brookfield-Methode untersucht. Die Ergebnisse sind in Tabelle 2 zusammengefaßt. Die Beispiele 5 bis 7 sind erfindungsgem**ä**ß die Beispiele V5 bis V7 dienen zum Vergleich. Die Beispiele und Vergleichsbeispiel zeigen, daß durch Zusatz von Esterquats zu den Methylmischethern die Viskositätsstabilität der Zubereitungen weiter verbessert werden kann. Des weiteren verleihen die Mittel bei Anwendung auf dem Haar diesem einen angenehmen Weichgriff und vermindern die elektrostatische Aufladung.

**Tabelle 2**

| **Viskositätsmessungen (Mengenangaben als Gew.-%)** | | | | | | |
|---|---|---|---|---|---|---|
| **Zusammensetzung/Performance** | **5** | **6** | **7** | **V5** | **V6** | **V7** |
| Cetearylalkohol+20 EO-Methylether | 1,3 | 0,5 | 0,5 | - | - | - |
| Dipalml Esterquat* | - | 0,8 | 0,8 | 1,3 | 0,8 | 0,8 |
| Ceteareth-20 | - | - | - | - | 0,5 | 0,5 |
| Cetearylalkohol | 3,0 | 3,0 | 3,0 | 3,0 | 3,0 | 3,0 |
| Coco Glycerides | - | - | 10,0 | - | - | 10,0 |
| Wasser | ad 100 | | | | | |

| ***Viskosität*** | | | | | | |
|---|---|---|---|---|---|---|
| ***- sofort, 20°C*** | 10.000 | 10.000 | 12.000 | 10.000 | 10.000 | 12.000 |
| ***- nach 4 w, 40°C*** | 9.000 | 10.000 | 12.000 | - | 7.000 | 9.000 |

| ***Stabilität*** | | | | | | |
|---|---|---|---|---|---|---|
| ***- sofort, 20°C*** | + | + | + | + | + | + |
| ***- nach 4 w, 40°C*** | + | + | + | - | + | + |

| | | | | | | |
|---|---|---|---|---|---|---|
| *) methylquatemiertes Difettsäuretriethanolaminester-Methosulfatsalz auf Basis teilgehärteter Palmfettsäure | | | | | | |

## Patentansprüche

1. Kosmetische und/oder pharmazeutische Zubereitungen, enthaltend
(a) Methylmischether und
(b) Esterquats.

2. Zubereitungen nach Anspruch 1, **dadurch gekennzeichnet,** daß sie Methylmischether der Formel **(I)** enthalten, 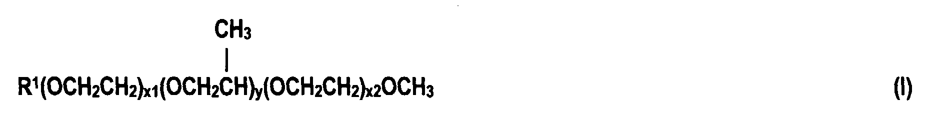 in der R¹ für einen linearen oder verzweigten Alkyl- und/oder Alkenylrest mit 6 bis 22 Kohlenstoffatomen, x1 und x2 unabhängig voneinander für 0 oder Zahlen von 1 bis 50, y für 0 oder Zahlen von 1 bis 5 steht, mit der Maßgabe, daß x und y nicht gleichzeitig für 0 stehen können.

3. Zubereitungen nach den Ansprüchen 1 und 2, **dadurch gekennzeichnet,** daß sie Esterquats der Formel **(II)** enthalten, 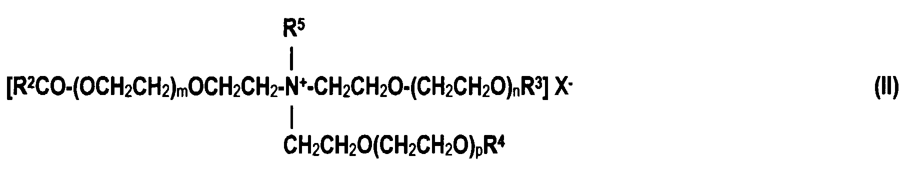 in der R²CO für einen Acylrest mit 6 bis 22 Kohlenstoffatomen, R³ und R⁴ unabhängig voneinander für Wasserstoff oder R²CO, R⁵ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

4. Zubereitungen nach den Ansprüchen 1 und 2, **dadurch gekennzeichnet,** daß sie Esterquats der Formel **(III)** enthalten, 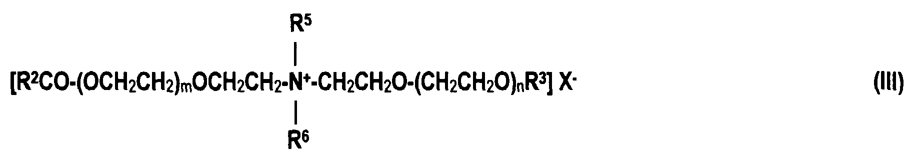 in der R²CO für einen Acylrest mit 6 bis 22 Kohlenstoffatomen, R³ für Wasserstoff oder R²CO, R⁵ und R⁶ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Zubereitungen nach den Ansprüchen 1 und 2, **dadurch gekennzeichnet,** daß sie Esterquats der Formel **(IV)** enthalten, 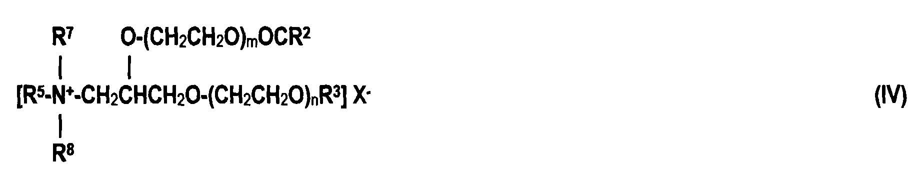 in der R²CO für einen Acylrest mit 6 bis 22 Kohlenstoffatomen, R³ für Wasserstoff oder R²CO, R³, R⁷ und R⁸ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

6. Zubereitungen nach den Ansprüchen 1 bis 5, **dadurch gekennzeichnet,** daß sie die Komponenten (a) und (b) im Gewichtsverhältnis 10 : 90 bis 90 : 10 enthalten.

7. Zubereitungen nach den Ansprüchen 1 bis 6, **dadurch gekennzeichnet,** daß sie die Komponenten (a) und (b) zusammen in Mengen von 0,1 bis 10 Gew.-% - bezogen auf die Mittel - enthalten.

8. Zubereitungen nach den Ansprüchen 1 bis 7, **dadurch gekennzeichnet,** daß sie Co-Emulgatoren enthalten, die ausgewählt sind aus der Gruppe, die gebildet wird von Anlagerungsprodukten von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; C_{12/18}-Fettsäuremono- und -diestern von Anlagerungsprodukten von 1 bis 30 Mol Ethylenoxid an Glycerin; Glycerinmono- und -diestern und Sorbitanmono- und -diestern von gesättigten und ungesättigten Fettsäuren mit 6 bis 22 Kohlenstoffatomen und deren Ethylenoxidanlagerungsprodukte; Alkylmono- und -oligoglycosiden mit 8 bis 22 Kohlenstoffatomen im Alkylrest und deren ethoxylierte Analoga; Anlagetungsprodukten von 15 bis 60 Mol Ethylenoxid an Ricinusöl und/oder gehärtetes Ricinusöl; Polyolestern; Anlagerungsprodukten von 2 bis 15 Mol Ethylenoxid an Ricinusöl und/oder gehärtetes Ricinusöl; Partialestern auf Basis linearer, verzweigter, ungesättigter bzw. gesättigter C_{6/22}-Fetts**äuren,** Ricinolsäure sowie 12-Hydroxystearinsäure und Glycerin, Polyglcerin, Pentaerythrit, Dipentaerythrit, Zuckeralkoholen, Alkylglucosiden sowie Polyglucosiden; Trialkylphosphaten sowie Mono-, Di- und/oder Tri-PEG-alkylphosphaten; Wollwachsalkoholen; Polysiloxan-Polyalkyl-Polyether-Copolymeren bzw. entsprechenden Derivaten; Mischestern aus Pentaeythrit, Fetts**äuren,** Citronensäure und Fettalkohol und/oder Mischester von Fettsäuren mit 6 bis 22 Kohlenstoffatomen, Methylglucose und Polyolen; Polyalkylenglycolen sowie Betainen.

9. Zubereitungen nach den Ansprüchen 1 bis 8, **dadurch gekennzeichnet,** daß sie Ölkörper enthalten, die ausgewählt sind aus der Gruppe, die gebildet wird von Guerbetalkoholen auf Basis von Fettalkoholen mit 6 bis 18, Estern von linearen C₆-C₂₂-Fettsäuren mit linearen C₆-C₂₂-Fettalkoholen, Estern von verzweigten C₆-C₁₃-Carbonsäuren mit linearen C₆-C₂₂-Fettalkoholen, Estern von linearen C₆-C₂₂-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, Estern von C₆-C₂₂-Fettalkoholen und/oder Guerbetalkoholen mit aromatischen Carbonsäuren, pflanzlichen Ölen, verzweigten primären Alkoholen, substituierten Cyclohexanen, linearen C₆-C₂₂-Fettalkoholcarbonaten, Guerbetcarbonaten, Dialkylethern, Ringöffnungsprodukten von epoxidierten Fettsäurealkylestern mit Polyolen, Siliconölen und/oder aliphatische bzw. naphthenische Kohlenwasserstoffen.

10. Verwendung von Methylmischethern nach Anspruch 2 als Emulgatoren zur Herstellung von kosmetischen und/oder pharmazeutischen Zubereitungen.
